# EUROPÄISCHE PATENTSCHRIFT

(11) **EP 2 545 890 B1**
(45) Veröffentlichungstag und Bekanntmachung des Hinweises auf die Patenterteilung: **12.04.2017**
(21) Anmeldenummer: 12188288.0
(22) Anmeldetag: 10.01.2006
(51) Int. Cl.: A61F 9/009, A61F 9/008

(54) **Sicherheitsmechanismus für ein Laserbehandlungsgerät**
Safety mechanism for a laser treatment unit
Mécanisme de sécurité pour une unité de traitement laser

(30) Priorität: 11.01.2005 DE 102005001249
(43) Veröffentlichungstag der Anmeldung: 16.01.2013
(62) Teilanmeldung aus: 06706186.1
(73) Patentinhaber: Carl Zeiss Meditec AG, 07745 Jena (DE)
(72) Erfinder: Mühlhoff, Dirk, 07751 Jena (DE); Lang, Carsten, 07607 Eisenberg (DE); Festag, Karsten, 07749 Jena (DE)
(74) Vertreter: Patentanwälte Geyer, Fehners & Partner mbB

(56) Entgegenhaltungen:
- EP-A- 0 589 825
- EP-A- 0 608 052
- DE-A1- 19 831 674
- US-A- 5 217 452

## Beschreibung

Die Erfindung bezieht sich auf ein Laserbehandlungsgerät für die Augenchirurgie, mit einem auf das Auge aufsetzbaren Kontaktglas, durch das ein Behandlungs-Laserstrahl fällt und einem Sicherheitsmechanismus, der eine Quetschung des Auges verhindert.

Solche Laserbehandlungsgeräte werden für laserchirurgische Verfahren am Auge angewendet. Dabei wird die Behandlungs-Laserstrahlung derart fokussiert, dass mittels eines optischen Durchbruchs eine Gewebeveränderung ausgelöst wird. Die Behandlungs-Laserstrahlung wirkt beispielsweise durch Photodisruption oder -ablation. Eine besonders vorteilhafte Anwendung dieses Prinzips findet sich bei der Fehlsichtigkeitskorrektur in der Ophthalmologie. Fehlsichtigkeiten des Auges rühren oftmals daher, dass die Brechungseigenschaften von Hornhaut und Linse keine ordnungsgemäße Fokussierung auf der Netzhaut bewirken. Bei Kurzsichtigkeit (auch als Myopie bezeichnet) liegt der Fokus bei entspanntem Auge vor der Netzhaut, bei Fernsichtigkeit (auch als Hyperopie bezeichnet) ist der Fokus dagegen hinter der Netzhaut. Eine Fehlsichtigkeit kann auch in Form eines Astigmatismus vorliegen, wenn die Fokussierung nicht in einem Brennpunkt, sondern linienförmig verzeichnet erfolgt.

Zur Fehlsichtigkeitskorrektur ist es bekannt, mittels Behandlungs-Laserstrahlen die Brechungseigenschaften der Hornhaut geeignet zu beeinflussen. Solche Verfahren sind beispielsweise in den US 5.984.916 und US 6.110.166 beschrieben. Dabei wird eine Vielzahl von optischen Durchbrüchen so aneinandergereiht, dass innerhalb der Hornhaut des Auges ein Teilvolumen isoliert wird. Dieses isolierte und somit vom übrigen Hornhautgewebe getrennte Teilvolumen wird dann über einen seitlich öffnenden Schnitt aus der Hornhaut herausgenommen. Die Gestalt des Teilvolumens ist dabei so gewählt, dass die Brechungseigenschaften der Hornhaut nach der Entnahme des Teilvolumens so geändert sind, dass die erwünschte Fehlsichtigkeitskorrektur erreicht ist.

Zur Ausbildung des Schnittes durch Aneinanderreihung von optischen Durchbrüchen ist es natürlich unumgänglich, die optischen Durchbrüche an exakt vorbestimmten Stellen zu erzeugen. Dies erfordert eine exakte Positionierung des Laserstrahls in der Augenhornhaut. Eine Verschiebung des Auges gegenüber dem Laserbehandlungsgerät muss deshalb so weit wie möglich vermieden oder ausgeglichen werden. Die US 6.373.571 und die WO 00/002008 A1 schlagen deshalb Kontaktlinsen vor, die als Adapter auf die Augenhornhaut aufgesetzt werden und diese gegenüber dem Laserbehandlungsgerät fixieren. Das Auge wird in der Regel mittels Unterdruck am Adapter festgesaugt. Dieser auch als Kontaktglas bezeichnete Adapter erfüllt zwei Funktionen: Zum einen verformt er das Auge entsprechend der durch ihn vorgegebenen Oberflächenform. Somit liegt eine definierte Oberflächenform im Strahlengang des Laserbehandlungsgerätes vor. Zum anderen fixiert das Kontaktglas das Auge und verhindert dadurch eine Verschiebung des Auges während des therapeutischen Eingriffs.

Um das Kontaktglas sicher am Auge auch bei Bewegungen des Patienten zu halten, schlägt die EP 0608052 A2 eine gattungsgemäße Vorrichtung vor, bei der das die Laserstrahlung fokussierende Objektiv gemeinsam mit dem Kontaktglas in einem Rahmen sitzt, der seinerseits federnd aufgehängt ist. Objektiv und Kontaktglas sind damit gemeinsam entlang der optischen Einfallachse der Behandlungs-Laserstrahlung verschieblich. Eine Bewegung des Patienten führt damit automatisch zu einer Verschiebung des Kontaktglases und der Fokussierungsoptik im Strahlengang. Diese Bewegung der Optik hat sich nun als nachteilig hinsichtlich der Qualität, mit der der Behandlungs-Laserstrahl fokussiert werden kann, herausgestellt.

Als Abhilfe dazu wäre es denkbar, das Kontaktglas und die Fokussierungsoptik dauerhaft und unverrückbar am Laserbehandlungsgerät zu befestigen. Dieser Ansatz birgt jedoch das Risiko, dass bei Bewegungen des Patienten das Auge durch Quetschung geschädigt wird. Eine solche Bewegung könnte entweder durch eine Körperbewegung des Patienten verursacht sein oder auftreten, wenn das Auge an das Kontaktglas angesetzt wird.

Der Erfindung liegt deshalb die Aufgabe zugrunde, ein Laserbehandlungsgerät der eingangs genannten Art so weiterzubilden, dass der Sicherheitsmechanismus eine Quetschung des Auges zuverlässig ausschließen kann, ohne sich negativ auf die optische Qualität des Laserbehandlungsgerätes auszuwirken.

Diese Aufgabe wird mit einem Laserbehandlungsgerät für die Augenchirurgie gemäß Anspruch 1 gelöst. Es hat eine Kopfanlage für einen Patienten und ein auf das Auge aufsetzbares Kontaktglas, durch das ein Behandlungs-Laserstrahl fällt, wobei ein Sicherheitsmechanismus vorgesehen ist, der bei einer entgegen der Laserstrahleinfallsrichtung auf das Kontaktglas gerichteten Kraft die Kopfanlage und das Kontaktglas auseinanderbewegt. Der Sicherheitsmechanismus ermöglicht ein Zurückweichen erst bei einer oberhalb eines Kraftgrenzwertes liegenden Kraft durch ein in Laserstrahleinfallsrichtung verstellbares Bett, das die Kopfanlage aufweist, wobei der Sicherheitsmechanismus zum Auseinanderbewegen das Bett verstellt und bei unterhalb des Kraftgrenzwertes liegender Kraft die Kopfanlage und das Kontaktglas fixiert.

Die Erfindung nimmt also von dem im Stand der Technik verfolgten Konzept, etwaige Augenbewegungen durch eine federnde Lagerung des Kontaktglases auszugleichen, grundlegend Abkehr und sieht ein in gewissen Rahmenbedingungen starres Kontaktglas vor. Somit sind optimale optische Verhältnisse bei der Beaufschlagung des Auges mit dem Behandlungs-Laserstrahl sichergestellt, und zugleich ist eine Quetschung des Auges ausgeschlossen, da der Kraftgrenzwert quasi eine Paniksicherung bewirkt.

Die Erfindung realisiert das Konzept, mittels eines Sicherheitsmechanismus eine Starrheit des Kontaktglases zu bewirken, die eine störende Defokussierung oder Fehlpositionierung der Behandlungs-Laserstrahlung bei Augenbewegungen bzw. Kopfbewegungen ausschließt. Die Starrheit ist dabei, wie erwähnt, strukturell hinsichtlich des Auges realisiert (Zurückweichen des Kontaktglases oberhalb eines Kraftgrenzwertes).
Es wird. Bei Quetschungsgefahr werden Kontaktglas und Patient auseinanderbewegt. Dieser Sachverhalt wird hier als Zurückweichen beschrieben. Darunter ist zu verstehen, dass der Patient vom Kontaktglas wegbewegt wird. Aus Sicht des Patienten ist das ein Zurückweichen des Kontaktglases, was die hier getroffene begriffliche Vereinheitlichung rechtfertigt.

Etwaige Ausgestaltungen und Weiterbildungen können selbstverständlich auch miteinander kombiniert werden.

In der Erfindung führt eine Erhöhung des Druckes, den der Patient, beispielsweise über sein Auge, auf das Laserbehandlungsgerät ausführt, erst dann zu einem Zurückweichen des Kontaktglases, wenn der Kraftgrenzwert überschritten ist. Eine Quetschung des Auges ist bei geeigneter Wahl des Kraftgrenzwertes ausgeschlossen, und zugleich ist bei normalen Verhältnissen ein optimaler Betrieb erreicht.

In einer besonders einfachen Bauweise wird der Kraftgrenzwert durch eine Feder- bzw. Gewichtskraft bewirkt. Eine Möglichkeit, dies zu erreichen, ist beispielsweise eine federnde Lagerung des Patienten auf einem Bett, die so gewählt ist, dass das Patientenbett bei einer scheinbaren Erhöhung des Patientengewichtes zurückweicht. Ein gesteigerter Druck des Auges auf das Kontaktglas wirkt sich in einer solchen scheinbaren Erhöhung des Patientengewichtes aus, so dass dann das gewünschte Zurückweichen eintritt. Das Laserbehandlungsgerät bzw. der optische Teil dieses Gerätes kann dabei räumlich fest bleiben. Anstelle der geschilderten mechanischen Ausgleichsmöglichkeit kann natürlich auch eine entsprechende Regelung, beispielsweise in Form einer elektronischen Regelung erfolgen.

In einer mechanisch einfacheren Bauweise ist es vorteilhaft, das Kontaktglas an einem Halteelement zu befestigen, das mit einer den Kraftgrenzwert definierten Kraft auf einen Anschlag des Gehäuses gedrückt ist. Bei einer den Kraftgrenzwert überschreitenden Andruckkraft gegenüber erfolgt eine Ausweichbewegung am Bett.

In einer vorteilhaften Weiterbildung der Erfindung kann die Laserbehandlung auch bei einem Zurückweichen des Kontaktglases fortgeführt werden, zumindest solange gewisse Rahmenbedingungen eingehalten sind. Dazu ist es zweckmäßig, wenn nicht nur das Kontaktglas zurückweicht, sondern auch die relevanten Teile der Optik, mit der der Behandlungslaserstrahl in das Auge oder auf das Auge fokussiert wird. Es ist deshalb bevorzugt, dass das Halteelement, an dem das Kontaktglas befestigt ist, auch eine Fokussieroptik, die den Behandlungs-Laserstrahl in oder auf das Auge fokussiert, trägt. Beim Zurückweichen bewegen sich dann Kontaktglas und Fokussieroptik gemeinsam.

Der Kraftgrenzwert ist zweckmäßigerweise so eingestellt, dass eine Quetschung des Auges sicher ausgeschlossen ist. Ein dafür geeigneter Wert liegt bei etwa 1 N.

Die der Erfindung eignet sich nicht nur dafür, dass eine Fehlverhalten des Patienten ohne Schaden bleibt, auch eine Gerätefehlfunktion kann abgefangen werden. Bei der Laserbehandlung wird der Patient auf einem Bett gelagert. Eine Höhenverstelleinrichtung erlaubt es, den Abstand zwischen Laserbehandlungsgerät bzw. dem Kontaktglas und dem Patienten zu verstellen. Der erfindungsgemäße Sicherheitsmechanismus verhindert zuverlässig, dass eine Fehlfunktion dieses Höhenverstellmechanismus eine Augenquetschung zur Folge hat. Bewegt beispielsweise der Höhenverstellmechanismus dem Patienten zu weit auf das Kontaktglas zu, sorgt der Sicherheitsmechanismus automatisch für ein Zurückweichen des Kontaktglases, bevor eine Quetschung des Auges zu befürchten ist.

In einer Weiterbildung der Erfindung ist eine Detektoreinrichtung vorgesehen, die bei einer einen Schwellwert überschreitenden Kontaktglasbewegung den Laserbehandlungsbetrieb sperrt. Der Schwellwert kann dabei nach verschiedenen Kriterien ausgewählt werden. Je nach Ausgestaltung des Laserbehandlungsgerätes kann der Schwellwert im Sinne einer Notabschaltung gewählt sein, die kurz vor Auftreten einer unzulässig großen Augenbelastung abschaltet, oder als qualitätssicherndes Merkmal dienen und an den durch die Bewegung verursachten optischen Fehlern orientiert sein.

Ist der Schwellwert so gewählt, dass er einen unzulässig hohen Augendruck verhindern soll, wird abgeschaltet, bevor das Zurückweichen des Kontaktglases an ein mechanisch bestimmtes Ende der Bewegung gelangt. Auch nach Überschreitung des Schwellwertes kann man dann noch Gegenmaßnahmen einleiten. Zum Beispiel kann ein Höhenverstellmechanismus des Patientenbettes abgeschaltet oder das Patientenbett schnell abgesenkt werden.

Eine Möglichkeit, das Überschreiten einer gewünschten Maximalbewegung zusätzlich zu detektieren, besteht darin, den Befestigungsmechanismus, mit dem das Auge am Kontaktglas fixiert wird, abzufühlen. Üblicherweise wird dafür Unterdruck verwendet. Die Detektoreinrichtung kann dann den Druck im Unterdrucksystem erfassen und so eine unzulässige Bewegung des Auges gegenüber dem Kontaktglas feststellen.

Aufgrund der Physiognomie des Menschen geht eine auf das Kontaktglas hin gerichtete Augenbewegung automatisch mit einer Kopfbewegung einher. Es ist deshalb möglich, die auf das Kontaktglas gerichtete Kraft nicht nur am Auge, sondern auch am Patientenkörper, vorzugsweise am Kopf, abzufühlen. Dieses Vorgehen stellt einen weiteren Schutz für das Auge dar. Es ist deshalb für alle genannten Varianten zweckmäßig, wenn eine Abstützeinrichtung vorgesehen ist, die eine am Patientenkörper anlegbare Stütze aufweist und die mit dem Sicherheitsmechanismus derart gekoppelt ist, dass eine bestimmte Kraft auf die Stütze entgegen der Laserstrahleinfallsrichtung ebenfalls ein Zurückweichen des Kontaktglases bewirkt. In der dritten Variante kann die Detektoreinrichtung einen Druck auf die Stütze erfassen.

Die Erfindung wird nachfolgend unter Bezugnahme auf die Zeichnungen beispielhalber noch näher erläutert. In den Zeichnungen zeigt:
- Fig. 1: eine schematische perspektivische Darstellung eines Laserbehandlungsgerätes zur Behandlung auf einem Patientenbett liegender Patienten,
- Fig. 2: eine schematische Teildarstellung des Strahlenganges des Laserbehandlungsgerätes der Fig. 1, gesehen entgegen der Blickrichtung des Patienten,
- Fig. 3: eine Darstellung des Strahlenganges der Fig. 2 in einer 90° gedrehten Ebene, d.h. aus Sichtweise eines hinter dem Patienten sitzenden Chirurgen,
- Fig. 4 und 5: Darstellungen eines Laserbehandlungsgerätes ähnlich dem der Fig. 3 in gleicher Ansicht wie in Fig. 3,
- Fig. 6 und 7: Darstellungen eines weiter modifizierten Laserbehandlungsgerätes in einer Ansicht ähnlich denen der Fig. 4 und 5,
- Fig. 8: eine schematische Darstellung des Laserbehandlungsgerätes der Fig. 1 in einer abgewandelten Bauweise in einer Darstellung ähnlich der der Fig. 3,
- Fig. 9: einen Gewichtsausgleichsmechanismus, der im Laserbehandlungsgerät der Fig. 1 vorgesehen ist,
- Fig. 10: eine schematische Darstellung eines Laserbehandlungsgerätes ähnlich dem der Fig. 1, allerdings in seiteninvertierter Darstellung mit einem zusätzlichen Schutzmechanismus, um einen Patienten vor Quetschungen zu schützen,
- Fig. 11: eine Ausschnittsvergrößerung der Fig. 10,
- Fig. 12: ein Diagramm zur Veranschaulichung der beim Betrieb eines Laserbehandlungsgerätes gemäß Fig. 1 auftretenden Kräfte am Auge,
- Figur 13: eine schematische Darstellung eines Schaltplanes für das Laserbehandlungsgerät der Fig. 1.

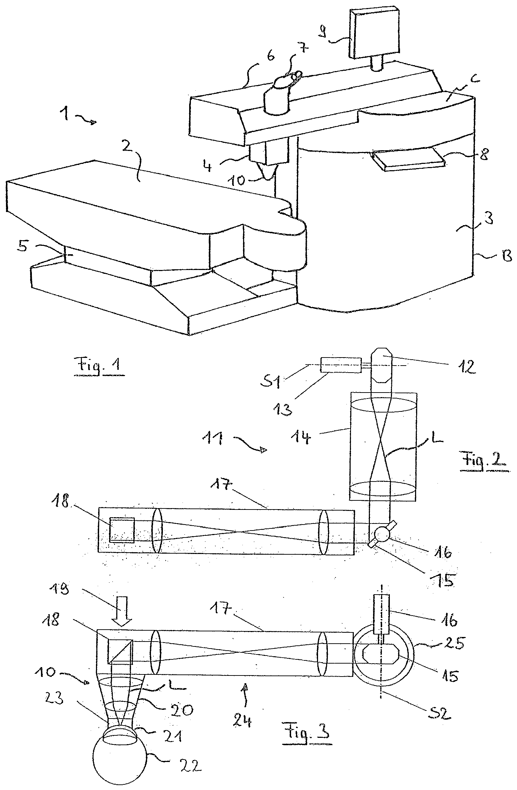

Die Fig. 1 zeigt ein Laserbehandlungsgerät in Form einer laserchirurgischen Behandlungsstation 1. Sie weist ein Bett 2 auf, auf dem ein (nicht gezeigter) Patient bei der Behandlung zu liegen kommt. Neben und über dem Bett ist eine Lasereinheit 3 angebracht, die einen Behandlungskopf 4 aufweist. Mittels einer am Bett 2 vorgesehenen Höhenverstelleinheit 5 kann der Abstand zwischen dem Bett 2 bzw. dem darauf liegenden Patienten und dem Behandlungskopf 4 verstellt werden. Der Behandlungskopf 4 befindet sich an einem Ausleger 6 der Lasereinheit 3, so dass er über dem Kopf eines Patienten ragt.

An einem am Ausleger 6 vorgesehener Mikroskop-Einblick 7 kann ein Chirurg den Behandlungsfortschritt verfolgen. Eine Tastatur 8 sowie ein Monitor 9 dienen dazu, Parameter des Laserbehandlungsverfahrens einzustellen. Die laserchirurgische Behandlungsstation 1 wird von einem Computer C gesteuert und ist zur ophtalmologischen Fehlsichtigkeitskorrektur gedacht.

Der Behandlungskopf 4 hat eine Spitze 10 an der ein Behandlungs-Laserstrahl austritt, und die zur Behandlung das Auge kontaktiert. Wie noch erläutert werden wird, ist der Behandlungskopf 4 mit der Spitze 10 im Ausleger 6 beweglich gelagert, so dass zusätzlich zu der durch die Höhenverstelleinheit 5 bewegten Verstellbarkeit weiterer Bewegungsspielraum zwischen der Spitze 10 und einem auf dem Bett 2 liegenden Patienten bzw. dessen Auge besteht.

Die Fig. 2 zeigt einen Ausschnitt des Behandlungsstrahlenganges 11, den die laserchirurgische Behandlungsstation 1 verwendet, um Behandlungs-Laserstrahlung L ins Auge des Patienten zu bündeln, dadurch optische Durchbrüche zu erzeugen und im Endeffekt eine Fehlsichtigkeitskorrektur auszuführen. Die Lasereinheit 3 verfügt über einen Laser, der die Behandlungs-Laserstrahlung L abgibt und einer die Behandlungs-Laserstrahlung L aufweitenden Aufweitungsoptik. Diese beiden Elemente sind für die hier zu erläuternde Sicherheitsfunktion der laserchirurgischen Behandlungsstation 1 nicht weiter von Relevanz und deshalb auch nicht in den Zeichnungen gezeigt. Die Aufweitungsoptik beinhaltet axial verschiebbare Elemente, so dass der Laserfokus in axialer Richtung in der Hornhaut verstellt werden kann.

Der Aufweitungsoptik nachgeschaltet ist ein erster Scanner mit einem Scanspiegel 12, der von einem Motor 13 angetrieben, um eine erste Ablenkachse S1 schwenkbar ist. Der erste Scanspiegel 12 liegt in einer Pupille eines noch zu erläuternden optischen Systems. Dem ersten Scanspiegel 12 folgt eine Pupillenabbildung 14, die sicherstellt, dass der erste Scanspiegel 12 in einer Pupille des optischen Systems liegt. In einer weiteren Pupille liegt ein zweiter Scanspiegel 15, der ebenfalls von einem Motor 16 angetrieben wird. Die Drehachse des zweiten Scanspiegel 15 steht senkrecht zur Ablenkachse S1 des ersten Scanspiegels 12. Es rotiert um eine zweite Ablenkachse S2, die in Figur 3 gestrichelt gezeigt. Die Ablenkachsen S1 und S2 der beiden Scanspiegel 12 und 15 stehen senkrecht aufeinander.

Dem zweiten Scanspiegel 15 ist eine Scanoptik 17 nachgeordnet, in deren Pupille der zweite Scanspiegel 15 liegt und deren Strahlengang von einem Strahlteiler 18 in die Spitze 10 umgelenkt wird. Die Spitze 10 enthält eine Fokussieroptik 20, die die Laserstrahlung L über ein Kontaktglas 23 in die Hornhaut 21 des Patientenauges 22 fokussiert. Der Strahlteiler 18 koppelt einen Beobachtungsstrahlengang 19 für den Mikroskop-Einblick 7 ein. Zugleich lenkt er den Strahlengang nach dem zweiten Scanspiegel 15 um 90° um.

Die Scanoptik 17, der Strahlteiler 18, die Fokussieroptik 20 und das Kontaktglas 23 bilden einen Arm 24. Der Arm 24 ist samt Motor 16 und Scanspiegel 15 an einem Drehgelenk 25 befestigt. Dadurch ist der Arm 24 um das Drehgelenk schwenkbar. Die Schwenkachse liegt in der Pupille, in der auch der Scanspiegel 15 angeordnet ist, und verläuft senkrecht zur Ablenkachse S2. Die Schwenkung des Armes 24 bewegt folglich das Kontaktglas 23 von der Augenhornhaut 21 weg.

Die Scanoptik der Ausbildung gemäß Fig. 2 und 3 ist an einem Träger 26 montiert und somit zum Arm 24 zusammengefaßt. Dieser Arm ist mit dem Drehgelenk 25 in Form eines Kugellagers verbunden. Die Achse dieses Kugellagers - es können der Stabilität wegen auch mehrere Lager auf einer gemeinsamen Achse verwendet werden - ist identisch mit der optischen Achse der vorausgehenden Pupillenabbildung 14. Beispielsweise kann man ein sehr großes Kugellager mit großem Durchmesser verwenden und dieses direkt auf die Fassung der Pupillenabbildung 14 aufstecken. Damit wird eine einfache Zentrierung des Drehgelenks 25 relativ zur optischen Achse der Pupillenabbildung 14 erreicht und die Schwenkachse liegt exakt in der Pupillenebene. Durch die hier vorgesehene, ansonsten natürlich optional zu verstehende Befestigung des zweiten Scanners 15 am Drehgelenk ist gewährleistet, dass die Ablenkachsen S1, S2 der beiden Scanner 12, 15 auch bei angehobenem Arm 24 senkrecht zueinander bleiben und trotzdem der vom zweiten Scanspiegel 15 reflektierte Strahl auch bei verschwenktem Arm 24 die Scanoptik 17 stets in vorbestimmter Richtung durchstrahlt.

Natürlich ist es alternativ möglich, auch die Pupillenabbildung 14 gemeinsam mit der Scanoptik 17, d.h. mit dem Arm 24, rotieren zu lassen. Auf diese Weise kann man eine große Länge der Führung der Drehachse realisieren, wodurch eine höhere Führungsgenauigkeit erreicht wird. In einer Weiterführung dieses Ansatzes rotiert die gesamte optische Einheit inklusive Laser. Im Sinne der Stabilität der gesamten optischen Anordnung ist eine solche Ausführung förderlich, allerdings steigen mit der Masse der gelagerten Einheit die Trägheitskräfte, die überwunden werden müssen, um ein Zurückweichen des Kontaktglases einzuleiten.

In einer weiteren Ausgestaltung ist eine Faserkopplung zwischen dem Laser und dessen Aufweitungsoptik verwendet. Dann sind alle restlichen Elemente der Optik auf der schwenkbaren Trägereinheit montiert. Vorteilhafterweise wird ein durch die Faser verursachter Chirp entweder vor Eintritt in die Faser oder danach durch eine Kompressoreinheit kompensiert. Vorzugsweise ist die Kompressoreinheit vor der Faser angeordnet, weil dadurch die Spitzenleistung in der Faser reduziert wird und eine lichtintensitätsbedingte Beschädigung der Faser vermieden wird. Gleichzeitig sinkt die Selbstphasenmodulation.

Die Bauweise der Fig. 2 und 3 in der laserchirurgischen Behandlungsstation 1 der Fig. 1 gestattet es dem Patienten, das Kontaktglas 23, welches z.B. mittels Unterdruck an seinem Auge befestigt wird, wegzudrücken. Das Kontaktglas 23 kann sich zusammen mit der Fokussieroptik 20 und der Scanoptik 17 vom Auge wegbewegen und dieses entlasten, um Quetschungen zu vermeiden. Mit der Masse der zu bewegenden Elemente kann die Initiierung der Bewegung allerdings eine Kraft erfordern, welche über das Auge des Patienten allein ohne Hilfsmittel nicht aufzubringen ist.

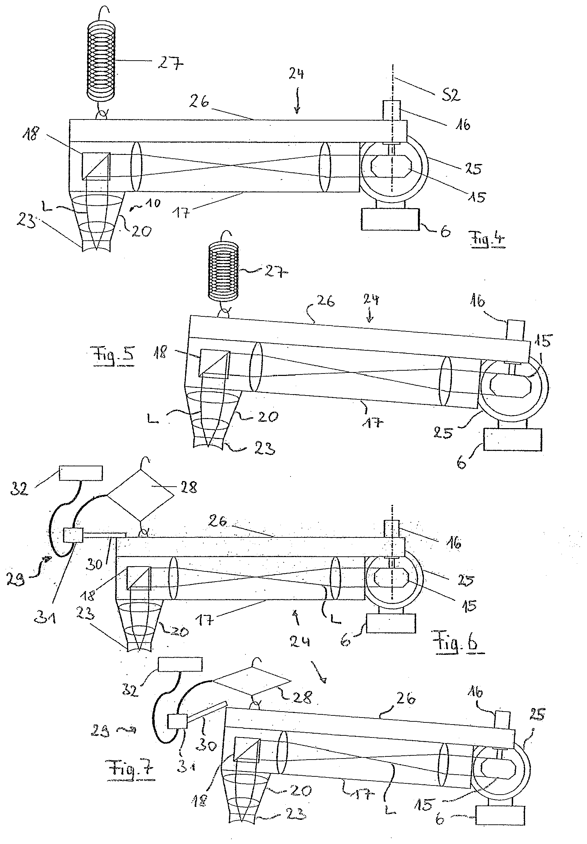

Es ist deshalb für massereiche optische Aufbauten eine Ausführungsform vorgesehen, wie sie in den Figuren 4 und 5 gezeigt ist. Dabei ist der Arm 24 mit dem Träger 26 versteift, an dem die Scanoptik 17 samt Strahlteiler 18 und Spitze 10 befestigt ist. Weiter greift am freien Ende des Träger 26 eine Feder-Aufhängung 27 an, welche die statischen Kräfte reduziert. Der Arm 24 bzw. Träger 26 ist weiter am Ausleger 6 so abgestützt, dass er mit einer definierten Kraft aufliegt. Diese Auflagekraft ist durch die Aufhängung 27 eingestellt.

Durch Druck auf das Kontaktglas 23 kann der Patient also mit einer vergleichsweise geringen Kraft den Arm 24 am Träger 26 von sich weg drücken, so dass er in die in Fig. 5 dargestellte angehobene Stellung gelangt. Lediglich die Auflagekraft ist zu überwinden. Die dabei erforderliche Kraft ist so eingestellt, dass eine Quetschung des Auges dabei nicht auftreten kann, beispielsweise beträgt die Kraft 1 N.

Fig. 5 zeigt weiter deutlich, dass sich der Scanspiegel 15 beim Verschwenken des Armes 24 mitdreht. Die Einkopplung der Laserstrahlung vom Scanspiegel 15 in die Scanoptik 17 bleibt damit auch bei ausgelenktem Träger 26 und damit angehobenen Kontaktglas 23 unverändert.

Der Aufbau der Fig. 4 und 5 kann allerdings dynamische Kräfte, die zum Einleiten der Armdrehung nötig sind, nicht kompensieren. Solche dynamischen Kräfte treten als Trägheitskräfte auf, wenn der Patient sich an das Kontaktglas heranbewegt, weil das Bett 2 nach oben gefahren wird. Zur Beschleunigung des Armes 24, die für ein Zurückweichen des Kontaktglases 23 erforderlich ist, ist eine zusätzliche Kraft erforderlich, die zumindest vorübergehend zu einer Stauchung des Auges führen kann. Will man diesen ab einem bestimmten Trägheitsmoment des am Drehgelenk 25 befestigten Armes 24 relativ großen Effekt vermeiden, ist es günstig eine Mechanik vorzusehen, die das Kontaktglas 23 aktiv zurückzieht, d.h. das Auge bei der Beschleunigung des Kontaktglases 23 am Arm 24 unterstützt. Dazu ist es für die hier beschriebene Bauweise nötig den Arm 24 aktiv anzuheben.

Die Fig. 5 und 6 zeigen ein Ausführungsbeispiel für eine solche Mechanik, die hier mittels Vakuum arbeitet. An das rotierbare Ende des Armes 24 ist am Träger 26 eine Vakuumdose befestigt. Herrscht in der Vakuumdose ein Unterdruck, so zieht sie sich zusammen und hebt den Träger 26 an seinem freien Ende an. Dieser Zustand ist in Fig. 7 dargestellt. Mittels eines Sensors 29, der hier als mechanischer Taster 30 ausgeführt ist, der einen Schalter 31 betätigt, wird eine Steuerung 32 eingeschaltet, sobald der Patient von unten liegend dem Arm 24 um ein gewisses Mindestmaß anhebt. Die Steuerung 23 aktiviert dann den Unterdruckantrieb 28, welcher den Träger 26 mit dem Arm 24 anhebt und damit das Kontaktglas 23 vom Auge wegzieht. Eine kleine Bewegung der Scanoptik führt also zur Betätigung des Unterdruckantriebs.

In einer Abwandlung kann auch nur ein Teil der Scanoptik oder an einer anderen Optik befestigtes zusätzliches Teil axial beweglich mit dem Rest der Scanoptik angebracht sein. Wird dieses Teil durch den Druck des Auges nach oben bewegt, so wird ein entsprechendes Signal für die Steuerung 32 abgeleitet, die wiederum den Unterdruckantrieb 28 in Gang setzt. Die dafür notwendige Ventilauslösung kann man dabei direkt mechanisch oder auch elektrisch vornehmen. Die Sensierung der Bewegung der Scanoptik kann natürlich auch kontaktlos, z.B. durch Lichtschranken oder kapazitive Abstandsensoren erfolgen.

Alternativ zum hier beschriebenen Unterdruckantrieb ist natürlich jeder geeignete Antrieb denkbar, beispielsweise auch mit elektrisch betriebenen Stellmotoren.

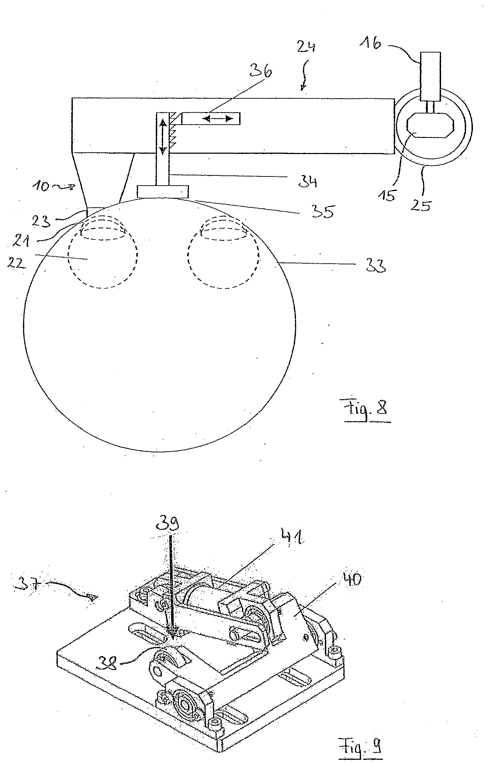

Anstelle oder zusätzlich zum aktiven Antreiben des Armes 24 kann eine Unterstützung im Wege eines mechanischen Abstandhalters verwendet werden, wie sie in Fig. 8 dargestellt ist. Der Abstandshalter weist einen am Kopf 33 des Patienten anlegbaren Stempel 34 auf, der bei aufgesetztem Kontaktglas 23 an die Stirn 35 des Patienten gelegt wird. Über einen Rastmechanismus wird der Stempel 34 dabei so eingestellt, dass er direkt an der Stirn 35 anliegt. Der Stempel erstreckt sich parallel zur Einstrahlungsrichtung, mit der die Laserbehandlungsstrahlung L durch die Spitze 10 auf das Kontaktglas 23 und die Hornhaut 21 fällt. Sobald die Hornhaut 21 am Kontaktglas 23 anliegt, wird der Stempel soweit nach unten gefahren, z.B. durch die eigene Gewichtskraft bewegt, dass er auf der Stirn des Patienten aufliegt. In dieser Position verriegelt er sich selbsttätig oder wird extern verriegelt. Bewegt sich nun das Auge 22 des Patienten nach oben, wird automatisch über den Stempel 34 der Arm 24 angehoben.

Zusätzlich oder alternativ zum Stempel 34 kann auch eine Abstützung direkt am Patientenbett 2 erfolgen. Damit wird ein unbeabsichtigtes Betätigen der Höhenverstelleinheit 5 sofort in ein Zurückziehen des Kontaktglases 23 durch Verschwenken des Armes 24 umgesetzt.

Es ist auch möglich, die Betätigung des Unterdruckantriebs 28 durch rein pneumatische Mittel zu bewirken. Der Taster 30 betätigt dann einen als Ventil ausgebildeten Schalter 31, der in einer Vakuumleitung zwischen einer Vakuumquelle, die in der Zeichnung der Steuerung 32 entspricht, und dem Unterdruckantrieb 28 liegt. Das Ventil wird geöffnet, wenn sich der Taster 30 nach oben bewegt, wie dies bei einer geringen Bewegung des Trägers 26 mit dem Arm 24 der Fall ist. Ist das Ventil offen, so wird der Unterdruckantrieb 28 evakuiert, zieht sich zusammen und kippt dadurch den Träger 26 mit dem Arm 24 nach oben.

Bei geeigneter Auslegung der im Arm 24 gehaltenen Optik genügt die Aufhängung 27, um eine Quetschung des Patientenauges zu vermeiden. Geht man von einer Armlänge von einem halben Meter aus und realisiert ein Trägheitsmoment des Armes 24 von 2 kg ∗ m², so führt eine Augenbewegung mit 6 mm pro Sekunde auf das Kontaktglas 23 hin bei einem Krümmungsradius von 7,8 mm und einem Krümmungsradius des Kontaktglases von 2 cm zu einer Kraft von 0,3 N, wenn das Auge beim Beschleunigen des Kontaktglases 23 um 0,77 mm eingedrückt wird. Das Kontaktglas 23 mit dem gesamten Arm 24 wird dann in einer Drittelsekunde auf die Bewegungsgeschwindigkeit des Auges beschleunigt. Es zeigt sich also, dass es bei einer geschickten Auslegung des Armes 24 ein externer Antrieb nicht zwingend erforderlich ist.

Fig. 9 zeigt eine mögliche Ausgestaltung für einen Federmechanismus, der die Funktion der Aufhängung 27 erfüllt. Es handelt sich dabei um eine Abstützmechanik 37, die den Arm 24 von unten her abstützt. Der Arm 24 liegt dabei auf einer Rolle 38 auf, die über einen Hebel 40 mit einer Feder 41 verbunden ist, die die Rolle 38 nach oben drückt. Die in Richtung des Pfeils 39 wirkende Gewichtskraft des Armes 24 kann durch geeignete Wahl bzw. Stellung der Feder 41 wunschgemäß bis auf eine Restauflagekraft kompensiert werden.

Für die geschilderten Bauweisen ist es völlig bedeutungslos, ob die auslösende Bewegung durch den Patienten oder durch eine Bewegung des Bettes 2 verursacht wird. Immer wird der Arm 24 angehoben.

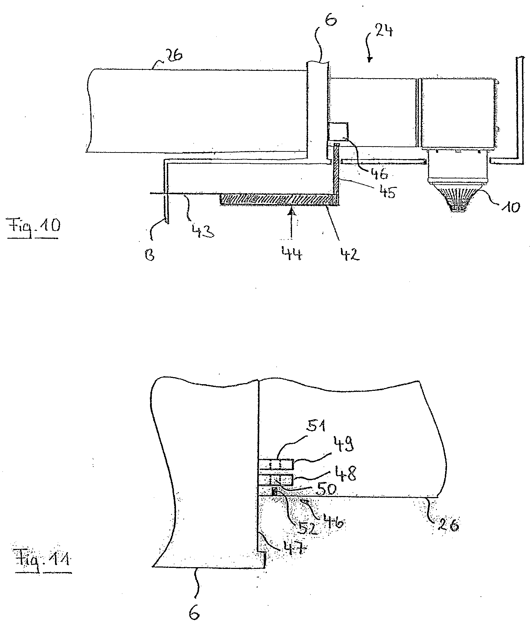

Fig. 10 zeigt eine weitere Detailansicht eines Auslegers 6 einer laserchirurgischen Behandlungsstation ähnlich der in Fig. 1 gezeigten Bauweise, wobei die Darstellung in Fig. 10 allerdings gegenüber der in Fig. 1 gewählten spiegelverkehrt ist. Wiederum ist zu sehen, dass der Arm 24 mit der Spitze 10 im Ausleger 6 vorgesehen ist, von dem lediglich Teile eines Gehäuses B gezeigt sind. Der Arm 24 ist mit dem Träger 26 gegenüber dem Ausleger 6 um einen in Fig. 10 links liegenden, aber nicht dargestellten Drehpunkt verschwenkbar. Bei dieser Verschwenkbewegung wird die Spitze 10 gegenüber dem Gehäuse B angehoben, so dass sie in das Gehäuse B hineinwandert. Der Arm 24 bzw. der Träger 26 liegt am Ausleger 6 an einer nicht gezeichneten Auflage auf. Ein Anheben des Auslegers 6 kann durch Krafteinwirkung an der Spitze 10 (am Kontaktglas 23) erfolgen.

Zusätzlich ist in der Bauweise der Fig. 10 noch ein Schutzmechanismus vorgesehen, der auch den Körper des Patienten vor Quetschungen durch den Arm 24 schützt. Dazu ist ein Prallblech 42 mittels eines Gelenkes 43, das beispielsweise als biegsame Befestigung in Form eines Stahlbleches ausgeführt sein kann, am Gehäuse B angebracht. Das Prallblech 42 stützt sich über einen Steg 45 am Arm 24 oder dessen Träger 26 ab. Eine in Richtung des Pfeils 44 auf das Prallblech 42 wirkende Kraft drückt dadurch nach oben auf den Arm 24. Ein Lagesensor 46 detektiert ein Anheben des Armes 24. Eine mögliche Ausführungsform für diesen Lagesensor 46, der die Verschiebung des Armes 24 gegenüber dem Gehäuse B bzw. dem Ausleger 6 erfaßt, ist in Fig. 11 exemplarisch gezeigt.

Wie Fig. 11 zu entnehmen ist, sind an einer Montagefläche 47 des Auslegers 6 gehäuseseitig Lichtschranken 48 und 49 angebracht, die Schlitze 50 und 51 aufweisen, durch die eine Positionsmarke 52 hindurchtreten kann, welche am Träger 26 bzw. dem Arm 24 angebracht ist. Ein Anheben des Armes 24 rückt somit die Positionsmarke 52 in den Schlitz 50 und ein weiteres Anheben auch in den Schlitz 51. Befindet sich die Positionsmarke 52 in Schlitz 50 bzw. 51 der Lichtschranke 48 bzw. 49, erzeugt diese ein entsprechendes Signal, das an eine (nicht gezeigte) Steuereinheit, beispielsweise den Computer C der laserchirurgischen Behandlungsstation 1 (vergl. Fig. 1), weiterleitet. Der Computer C steuert dann eine entsprechende Gegenreaktion des Systems, beispielsweise ein Abschalten der Behandlungs-Laserstrahlung L oder ein Absenken des Patientenbettes 2.

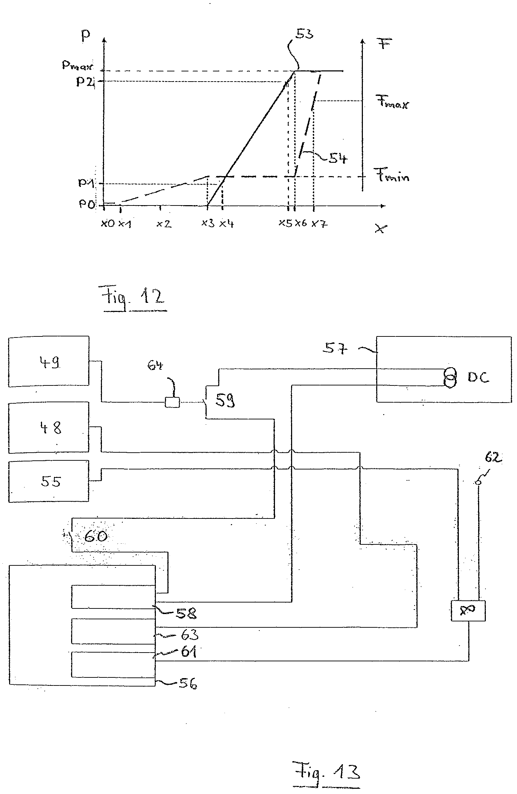

Fig. 12 zeigt schematisch einen beispielhaften Zusammenhang zwischen der Position P des Armes 24 bzw. der Spitze 10 der laserchirurgischen Behandlungsstation 1 bzw. der Kraft F auf das Auge des Patienten, jeweils als Funktion der Augenposition x, die bei einem auf dem Bett 2 liegenden Patienten durch die Position der Höhenverstelleinheit 5 vorgegeben ist. Wird ein Patient für die Behandlung vorbereitet, bringt man zuerst ein neues, steriles Kontaktglas 23 an der Spitze 10 an. Dann wird der Patient auf das Bett 2 gelegt, dessen Höhenverstelleinheit 5 vom Chirurgen an der laserchirurgischen Behandlungsstation 1 gesteuert wird. Der Computer C weist dazu eine geeignete Eingabevorrichtung, beispielsweise einen Joystick, auf und steuert die Höhenverstelleinheit 5 entsprechend an. Zu Beginn ist die Höhenverstelleinheit 5 nach unten gefahren, woraus sich der Ort x0 ergibt. Zugleich liegt die Spitze 10 an ihrer tiefsten Position P0, da der Arm 24 im Ausleger 6 am unteren Anschlag anliegt. Nun fährt der Chirurg den Patienten mittels der Höhenverstelleinheit 5 nach oben, bis am Ort x1 das Auge des Patienten in Kontakt mit dem Kontaktglas 23 tritt. Der Chirurg verfährt den Patienten nun weiter langsam nach oben, bis das Auge vollständig am Kontaktglas 23 anliegt. Dies ist beim Ort x2 der Fall, der dadurch gekennzeichnet ist, dass der Unterdruck, der das Kontaktglas 23 an der Hornhaut 21 befestigt, angelegt werden kann.

Damit die Hornhaut 21 möglichst vollständig an der Innenfläche des Kontaktglases 23 anliegt, drückt das Auge 23 mit einer gewissen Kraft gegen das Kontaktglas 23. Da diese Kraft jedoch noch geringer als die Kraft Fmin ist, bei der der Arm 24 angehoben wird, bleibt der Arm 24 dabei noch ruhend liegen.

Mit dem Einschalten des Unterdrucks hebt der Computer C automatisch die Höhenverstelleinheit 5 noch etwas an, so dass das Bett 2 noch etwas über den Ort x2 angehoben wird, um einen sicheren Verschluß des Kontaktglases 23 mittels Unterdruck an der Hornhaut 21 zu gewährleisten. Die Höhenverstelleinheit 5 bzw. der Kopf des Patienten befindet sich somit zwischen den Orten x2 und x3. Das Auge drückt mit einer unter der minimalen Kraft Fmin liegenden Kraft gegen das Kontaktglas 23, so dass der Arm 24 immer noch in der Position P0 verbleibt, also nicht ausgehoben wird. Das Auge ist am Kontaktglas fixiert, und die Behandlung kann begonnen werden.

Bewegt sich während der Behandlung der Kopf des Patienten nach oben, beispielsweise, weil der Patient den Kopf bewegt, oder wegen einer ungewollten Betätigung der Höhenverstelleinheit 5, so ist erst am Ort x3 die die Kraft auf den Arm 24 gleich der Mindestkraft Fmin, mit der der Arm 24 im Ausleger 6 aufliegt. Bei einer weiteren Aufwärtsbewegung des Kopfes, wird der Ausleger 24 angehoben. Dieser Fall entspricht der in Fig. 12 steigenden Ortskurve 53 (mit durchgezogener Linie dargestellt), und der Arm 24 verläßt die Ruheposition P0. Ist der Ausleger in der Position P1 angelangt, weil der Kopf des Patienten bzw. im Falle einer Fehlfunktion oder Fehlbedienung die Höhenverstelleinheit 5 den Ort x4 erreichte, gibt die erste Lichtschranke 48 ein Schaltsignal ab. Dadurch, dass der Arm 24 mit der eingestellten Kraft Fmin ausgehoben werden kann, steigt dabei die auf das Auge ausgeübte Kraft und damit der Druck auf das Auge nicht weiter an.

Das bei Position P1 erreichte Schaltsignal veranlaßt den Computer C den Laserstrahl L so zu schalten, dass keine Behandlung mehr erfolgt. Beispielsweise kann der Laser abgeschaltet werden oder die Laserstrahlenergie so reduziert werden, dass keine optischen Durchbrüche mehr entstehen. Darüber hinaus ist es möglich, eine Warnung für den Chirurgen auszugeben, beispielsweise in Form einer entsprechenden Anzeige am Monitor 9.

Schließlich kann man im Computer C einen Schaltmechanismus vorsehen, der bei Erreichen der Position P1 automatisch die Höhenverstelleinheit 5 nach unten, d.h. zu kleineren x-Werten bewegt, um das Auge wieder in den normalen Behandlungsbereich zwischen x2 und x3 zurückzuführen. Ist dies gelungen, wechselt das Schaltsignal der Lichtschranke 48 wieder in den Ruhestand, der normale Behandlungsbetrieb wird wieder aufgenommen und die Warnung zurückgenommen. Bewirkt man die Relativbewegung von Auge und Kontaktglas nur durch Bewegung des Bettes, kann der Schaltmechanismus auf die x-Werte abgestimmt werden, so dass z.B. eine Bewegung bei Erreichen von x3 erfolgt.

Bewegt sich jedoch durch eine Fehlfunktion oder eine entsprechende Aktion des Patienten der Arm 24 weiter nach oben und erreicht die Position P2, so spricht die zweite Lichtschranke 49 an und der Computer C leitet dann daraufhin eine Notabschaltung ein, die zum einen die Höhenverstelleinheit 5 deaktiviert und nach unten fährt und zum anderen die laserchirurgische Behandlungsstation 1 bis auf die Steuerung abschaltet. Dies geschieht, um zu verhindern, dass über den Ort x5 hinaus der Ort x6 erreicht wird, an dem der Arm 24 an seiner maximalen Auslenkung in der Position Pmax ankommt, an der ein weiteres Zurückweichen nicht möglich ist. Würde die anhebende Bewegung des Kopfes dann noch anhalten, stiege ab dem Ort x6 die Kraft auf die Hornhaut 21 bzw. das Auge 22 sprunghaft an, wie die Kraftkurve 24 der Fig. 12 deutlich zeigt. Am Ort x7 wäre die maximal zulässige Kraft Fmax auf das Auge 22 erreicht und es bestünde die Gefahr der Quetschung.

Durch die am Ort x5 bzw. der Position P2 erfolgte Notabschaltung der laserchirurgischen Behandlungsstation 1 ist die Quetschung des Auges 22 auch bei einem panikartigen Verhalten des Patienten vermieden.

Da in Ausführungsform gemäß Fig. 10 das Prallblech 42 unterhalb des Auslegers 6 liegt, ist auch eine Quetschung des Patientenkörpers vermieden, die eintreten könnte, wenn die Höhenverstelleinheit 5 den Patienten gegen den Ausleger 6 drückte.

Fig. 13 zeigt eine Schaltung, die z.B. vom Computer C realisiert sein kann, um das anhand Fig. 12 geschilderte Schutzverfahren auszuführen. In Fig. 13 sind die exemplarischen Lichtschranken 48 und 49 der Fig. 11 allgemein als Sensoren gezeichnet, die erfassen, ob der Arm 24 in die Positionen P1 bzw. P2 gelangt ist. Weiter ist in Fig. 13 schematisch ein Ansaugdrucksensor 55 eingezeichnet, der überprüft, ob der Unterdruck, welcher zur Ansaugung des Kontaktglases 23 verwendet wird, in einem Wertebereich ist, bei dem eine zuverlässige Ansaugung des Auges 22 an das Kontaktglas 23 gegeben ist. Die Sensoren sowie der Ansaugdrucksensor 55 wirken auf noch zu beschreibende Weise auf einen Antrieb 56 der Höhenverstelleinheit 5. Der Antrieb 56 wird von einer Gleichstromquelle 57 versorgt, die eine Stromversorgung 58 des Antriebes 56 speist. Die Stromquelle 57 ist mit zwei Leitungen mit der Stromversorgung 58 verbunden. In eine Zuleitung sind zwei Notschalter 59 und 60 geschaltet, die bei Aktivierung öffnen und im nicht betätigten Zustand geschlossen sind.

Der Notschalter 59 wird von der zweiten Lichtschranke 49 angesteuert und der Notschalter 60 dient dem Chirurgen als mechanischer Notschalter, so dass jederzeit die Verbindung zwischen Stromquelle 57 und Stromversorgung 58 des Antriebs 56 unterbrechen und damit den Antrieb 56 stillegen kann.

Der Antrieb 56 weist weiter einen Sperrmechanismus 61 auf, der bei Aktivierung den Antrieb 56 abschaltet. Diese Sperrung erfolgt, wenn sowohl ein Unterdruckfühler 62 anzeigt, dass die Ansaugung des Auges 22 an das Kontaktglas 23 eingeschaltet ist, als auch der Ansaugdrucksensor 55 ein angesaugtes Auge signalisiert. In diesem Zustand verhindert der Sperrmechanismus 61 jegliche weitere Verstellung der Höhenverstelleinheit 5 über den Antrieb 56, da bei angesaugtem Auge eine Verstellung der Höhenverstelleinheit 5 nicht erforderlich und kann sogar schädlich sein kann.

Im Antrieb 56 ist weiter ein Blockiermechanismus 63 vorgesehen, der von der ersten Lichtschranke 48 angesteuert wird und parallel zum Sperrmechanismus 61 jegliche Aktivität des Antriebs 56 unterbindet, wenn die erste Lichtschranke 58 anzeigt, dass der Arm 24 in die Position P1 gelangte. Damit wird verhindert, dass die Höhenverstelleinheit 5 versehentlich betätigt wird und den Patienten anhebt, was möglich wäre, falls durch eine Bewegung des Patienten der Unterdruck abreißt und der Ansaugdrucksensor 55 dadurch nicht mehr signalisiert, dass das Auge korrekt angesaugt ist. Dadurch ist bei einer beispielsweise seitwärts und aufwärts führenden Bewegung des Patienten ebenfalls ein Betrieb des Antriebs 56 und damit eine Verstellung der Höhenverstelleinheit 5 unterbunden.

Das parallele Vorsehen des Sperrmechanismus 61 sowie des Blockiermechanismus 63 erlaubt es dadurch, mittels der Höhenverstelleinheit 5 eine Regelung auszuführen, die ein sicheres Ansaugen des Auges garantiert.

Die zweite Lichtschranke 59, die ein Signal abgibt, wenn sich der Arm in der Position P2 befindet, ist über ein Relais 64 mit dem Notschalter 59 verbunden. Gibt die zweite Lichtschranke 59 ein die Position P2 anzeigendes Signal ab, wird der Notschalter 59 geöffnet und der Antrieb 56 stromlos geschaltet. Je nach Gestaltung des Antriebes 56 verharrt das Bett 2 dann in der gegenwärtigen Höheneinstellung oder gleitet sanft nach unten.

Das beschriebene erfindungsgemäße System vermeidet Quetschungen am Auge bei einer laserchirurgischen Behandlungsstation, indem die am Auge aufliegende Komponente automatisch eine Ausweichbewegung ausführt, falls der Patient angehoben wird oder den Kopf hebt. Zugleich ist die Ausweichbewegung vorteilhafterweise so realisiert, dass die optische Qualität der Behandlung während des Ausweichens möglichst unverändert bleibt. Darüber hinaus ist über entsprechende Sensoren und Steuerungsmechanismen sichergestellt, dass eine zu einer Quetschung des Auges führende Bewegung nicht auftreten kann.

Die Erfindung kann folgendermaßen charakterisiert werden:
1. Laserbehandlungsgerät für die Augenchirurgie, mit einer Kopfanlage (2) für einen Patienten und einem auf das Auge (21) aufsetzbaren Kontaktglas (23), durch das ein Behandlungs-Laserstrahl (L) fällt, wobei ein Sicherheitsmechanismus (24, 25) vorgesehen ist, der bei einer entgegen der Laserstrahleinfallsrichtung auf das Kontaktglas (23) gerichteten Kraft die Kopfanlage (2) und das Kontaktglas (23) auseinanderbewegt,
   dadurch gekennzeichnet, dass der Sicherheitsmechanismus (24, 25) das Auseinanderbewegen erst bei einer oberhalb eines Kraftgrenzwertes (Fmin) liegenden Kraft ermöglicht und bei unterhalb des Kraftgrenzwertes liegender Kraft die Kopfanlage (2) und das Kontaktglas (23) zueinander fixiert.
2. Laserbehandlungsgerät nach Nr. 1, dadurch gekennzeichnet, dass der Sicherheitsmechanismus (24, 25) das Kontaktglas (23) zurückzieht.
3. Laserbehandlungsgerät nach Nr. 1 oder 2, dadurch gekennzeichnet, dass der Kraftgrenzwert (Fmin) durch eine Feder- und/oder Gewichtskraft bewirkt ist.
4. Laserbehandlungsgerät nach Nr. 1, 2 oder 3, dadurch gekennzeichnet, dass das Kontaktglas (23) an einem Halteelement (24) befestigt ist, das mit einer den Kraftgrenzwert (Fmin) definierenden Kraft auf einen Anschlag des Gehäuses (B, 6) gedrückt ist.
5. Laserbehandlungsgerät nach Nr. 4, dadurch gekennzeichnet, dass das Halteelement (24) auch eine Fokussieroptik (20), die den Behandlungs-Laserstrahl (L) in oder auf das Auge (21) fokussiert, trägt.
6. Laserbehandlungsgerät nach einer der obigen Nummern, dadurch gekennzeichnet, dass der Kraftgrenzwert 1 Newton beträgt.
7. Laserbehandlungsgerät für die Augenchirurgie, mit
   - einer Kopfanlage (2) für einen Patienten,
   - einer Strahlablenkeinheit (15, 16), die einen Behandlungs-Laserstrahl (L) mindestens um eine Achse (S2) variabel ablenkt,
   - einer Fokussieroptik (20), die der Strahlablenkeinheit (15, 16) nachgeordnet ist, die den Laserstrahl (L) längs einer optischen Achse in oder auf das Auge (21) fokussiert,
   - einem auf das Auge (21) aufsetzbaren Kontaktglas (23), das der Fokussieroptik (20) nachgeordnet, und
   - einem Sicherheitsmechanismus (24, 25), der bei einer entgegen der Laserstrahleinfallsrichtung auf das Kontaktglas (23) gerichteten Kraft die Kopfanlage (2) und das Kontaktglas (23) auseinanderbewegt,
   dadurch gekennzeichnet, dass
   - die Strahlablenkeinheit (15, 16) zumindest hinsichtlich eines für die eine Achse (S2) der Ablenkung wirkenden Ablenkelementes (15) in der Eintrittspupille der Fokussieroptik (20) angeordnet ist und
   - der Sicherheitsmechanismus (24, 25) das Kontaktglas (23), die Fokussieroptik (20) und das Ablenkelement (15) so koppelt, dass beim Auseinanderbewegen das Ablenkelement (15) in der Eintrittspupille bleibt und die Länge des Lichtweges zwischen dem Ablenkelement (15) und dem Kontaktglas (23) konstant ist.
8. Laserbehandlungsgerät nach Nr. 7, dadurch gekennzeichnet, dass der Sicherheitsmechanismus (24, 25) das Kontaktglas (23) zurückzieht.
9. Laserbehandlungsgerät nach Nr. 8, dadurch gekennzeichnet, dass das Kontaktglas, die Fokussieroptik und das Ablenkelement zu einer Einheit starr verbunden sind und der Sicherheitsmechanismus eine Längsführung dieser Einheit aufweist.
10. Laserbehandlungsgerät nach Nr. 8 oder 9, dadurch gekennzeichnet, dass der Lichtweg des Laserstrahls (L) nach der Eintrittspupille der Fokussieroptik (20) mindestens einmal umgelenkt ist (18) und der Sicherheitsmechanismus (24, 25) beim Zurückziehen eine gemeinsame Dreh- bzw. Schwenkbewegung von Kontaktglas (23), Fokussieroptik (20) und Ablenkelement (15) bewirkt.
11. Laserbehandlungsgerät nach Nr. 10, dadurch gekennzeichnet, dass das Kontaktglas (23), die Fokussieroptik (20) und das Ablenkelement (15) zu einem Arm starr verbunden sind und der Sicherheitsmechanismus eine Drehlagerung (25) des Armes mit Drehachse in der Eintrittspupillenebene aufweist.
12. Laserbehandlungsgerät nach einer der Nummern 8 bis 11, dadurch gekennzeichnet, dass der Sicherheitsmechanismus (24, 25) eine Gewichtskraftkompensationseinrichtung (27, 28) aufweist, insbesondere in Form eines Gegengewichtes oder eines Federelementes (27, 41).
13. Laserbehandlungsgerät nach Nr. 12 in Verbindung mit einer der Nummern 1 bis 6, dadurch gekennzeichnet, dass die Gewichtskraftkompensationseinrichtung (27, 28) den Kraftgrenzwert einstellt.
14. Laserbehandlungsgerät nach Nr. 11 in Verbindung mit einer der Nummern 1 bis 6, dadurch gekennzeichnet, dass der Arm mit dem Kraftgrenzwert (Fmin) am Gehäuse (B) des Laserbehandlungsgerätes (1) abgestützt ist.
15. Laserbehandlungsgerät nach einer der Nummern 7 bis 14, gekennzeichnet durch eine Abstützeinrichtung (34, 36), die eine am Patientenkörper (33) anlegbare Stütze (34) aufweist und die mit dem Sicherheitsmechanismus (24, 25) derart gekoppelt ist, dass eine bestimmte Kraft auf die Stütze (34) entgegen der Laserstrahleinfallsrichtung ebenfalls das Auseinanderbewegen bewirkt.
16. Laserbehandlungsgerät nach einer der obigen Nummern, gekennzeichnet durch ein in Laserstrahleinfallsrichtung verstellbares Bett (2), das die Kopfanlage aufweist, wobei der Sicherheitsmechanismus zum Auseinanderbewegen das Bett (2) verstellt.
17. Laserbehandlungsgerät für die Augenchirurgie, mit einem auf das Auge (21) aufsetzbaren Kontaktglas (23), durch das ein Behandlungs-Laserstrahl (L) fällt, und einem Sicherheitsmechanismus (24, 25), der das Kontaktglas (23) beweglich hält, so dass es bei einer entgegen der Laserstrahleinfallsrichtung auf das Kontaktglas (23) gerichteten Kraft zurückweicht,
   dadurch gekennzeichnet, dass der Sicherheitsmechanismus (24, 25) eine Detektoreinrichtung (29, 46) aufweist, welche ein Zurückweichen des Kontaktglases (23) überwacht und bei einer einen Schwellwert (P1) überschreitenden Kontaktglasbewegung einen Laserbehandlungsbetrieb des Laserbehandlungsgerätes (1) unterbricht.
18. Laserbehandlungsgerät nach einer der obigen Nummern, dadurch gekennzeichnet, dass der Sicherheitsmechanismus einen Antrieb (28, 5) zum aktiven Auseinanderbewegen von Kontaktglas (23) und Auge (21) aufweist und eine Steuereinrichtung (C) bei einer den Kraftgrenzwert (Fmin) überschreitenden Kraft bzw. einer den Schwellwert (P1) überschreitenden Kontaktglasbewegung den Antrieb (28, 5) zum aktiven Auseinanderbewegen ansteuert.
19. Laserbehandlungsgerät nach den Nr. 18 und 10, dadurch gekennzeichnet, dass der Antrieb (28) eine Schwenk- oder Drehbewegung bewirkt.
20. Laserbehandlungsgerät nach den Nr. 18 und 11, dadurch gekennzeichnet, dass der Antrieb (28) den Arm dreht.
21. Laserbehandlungsgerät nach den Nr. 15 und 17, dadurch gekennzeichnet, dass die Detektoreinrichtung einen Druck auf die Stütze (34) erfaßt.
22. Laserbehandlungsgerät nach den Nr. 16 und 17, dadurch gekennzeichnet, dass der Sicherheitsmechanismus bei der den Schwellwert (P1) überschreitenden Kotaktglasbewegung zusätzlich das Bett (2) absenkt.

## Patentansprüche

1. Laserbehandlungsgerät für die Augenchirurgie, mit einer Kopfanlage (2) für einen Patienten und einem auf das Auge (21) aufsetzbaren Kontaktglas (23), durch das ein Behandlungs-Laserstrahl (L) fällt, wobei ein Sicherheitsmechanismus (24, 25) vorgesehen ist, der dazu ausgebildet ist bei einer entgegen der Laserstrahleinfallsrichtung auf das Kontaktglas (23) gerichteten Kraft die Kopfanlage (2) und das Kontaktglas (23) auseinanderzubewegen,
**dadurch gekennzeichnet, dass** der Sicherheitsmechanismus dazu ausgebildet ist das Auseinanderbewegen bei einer oberhalb des Kraftgrenzwertes (Fmin) liegenden Kraft durch ein in Laserstrahleinfallsrichtung verstellbares Bett (2), das die Kopfanlage aufweist zu ermöglichen, wobei der Sicherheitsmechanismus dazu ausgebildet ist zum Auseinanderbewegen das Bett (2) zu verstellen und bei unterhalb des Kraftgrenzwertes liegender Kraft die Kopfanlage (2) und das Kontaktglas (23) zueinander zu fixierten.

2. Laserbehandlungsgerät nach Anspruch 1, **dadurch gekennzeichnet, dass** der Sicherheitsmechanismus dazu ausgebildet ist das Kontaktglas (23) zurückzuziehen.

3. Laserbehandlungsgerät nach Anspruch 1 oder 2, **dadurch gekennzeichnet, dass** der Kraftgrenzwert (Fmin) durch eine Feder- und/oder Gewichtskraft bewirkt ist.

4. Laserbehandlungsgerät nach Anspruch 1, 2 oder 3, **dadurch gekennzeichnet, dass** das Kontaktglas (23) an einem Halteelement (24) befestigt ist, das mit einer den Kraftgrenzwert definierenden Kraft auf einen Anschlag des Gehäuses (B, 6) gedrückt ist.

5. Laserbehandlungsgerät nach Anspruch 4, **dadurch gekennzeichnet, dass** das Halteelement (24) auch eine Fokussieroptik (20), die den Behandlungs-Laserstrahl (L) in oder auf das Auge (21) fokussiert, trägt.

6. Laserbehandlungsgerät nach einem der obigen Ansprüche, **dadurch gekennzeichnet, dass** der Kraftgrenzwert 1 Newton beträgt.

7. Laserbehandlungsgerät nach Anspruch 1, mit einer Strahlablenkeinheit (15, 16), die den Behandlungs-Laserstrahl (L) mindestens um eine Achse (S2) variabel ablenkt, und einer Fokussieroptik (20), die der Strahlablenkeinheit (15, 16) nachgeordnet ist, die den Laserstrahl (L) längs einer optischen Achse in oder auf das Auge (21) fokussiert, wobei das Kontaktglas (23) der Fokussieroptik (20) nachgeordnet ist.

8. Laserbehandlungsgerät nach Anspruch 7, **dadurch gekennzeichnet, dass** der Sicherheitsmechanismus das Kontaktglas (23) zurückzieht.

9. Laserbehandlungsgerät nach Anspruch 8, **dadurch gekennzeichnet, dass** das Kontaktglas (23), die Fokussieroptik (20) und die Strahlablenkeinheit (15, 16) zu einer Einheit starr verbunden sind und der Sicherheitsmechanismus eine Längsführung dieser Einheit aufweist.

10. Laserbehandlungsgerät nach Anspruch 8 oder 9, **dadurch gekennzeichnet, dass** der Lichtweg des Laserstrahls (L) nach der Eintrittspupille der Fokussieroptik (20) mindestens einmal umgelenkt ist (18) und der Sicherheitsmechanismus beim Zurückziehen eine gemeinsame Dreh- bzw. Schwenkbewegung von Kontaktglas (23), Fokussieroptik (20) und Ablenkelement (15) bewirkt.

11. Laserbehandlungsgerät nach Anspruch 10, **dadurch gekennzeichnet, dass** das Kontaktglas (23), die Fokussieroptik (20) und das Ablenkelement (15) zu einem Arm starr verbunden sind und der Sicherheitsmechanismus eine Drehlagerung (25) des Armes mit Drehachse in der Eintrittspupillenebene aufweist.

12. Laserbehandlungsgerät nach einem der Ansprüche 8 bis 11, **dadurch gekennzeichnet, dass** der Sicherheitsmechanismus eine Gewichtskraftkompensationseinrichtung (27, 28) aufweist, insbesondere in Form eines Gegengewichtes oder eines Federelementes (27, 41).

13. Laserbehandlungsgerät nach Anspruch 12 **dadurch gekennzeichnet, dass** die Gewichtskraftkompensationseinrichtung den Kraftgrenzwert einstellt.

14. Laserbehandlungsgerät nach einem der Ansprüche 7 bis 13, **gekennzeichnet durch** eine Abstützeinrichtung (34, 36), die eine am Patientenkörper (33) anlegbare Stütze (34) aufweist und die mit dem Sicherheitsmechanismus derart gekoppelt ist, dass eine bestimmte Kraft auf die Stütze (34) entgegen der Laserstrahleinfallsrichtung ebenfalls das Auseinanderbewegen bewirkt.

## Claims

1. A laser treatment apparatus for ophthalmic surgery with a headrest (2) for a patient and a contact glass (23), which can be placed onto the eye (21) and through which a treatment laser beam (L) is incident; wherein a safety mechanism (24, 25) is provided which is adapted to move the headrest (2) and the contact glass (23) apart when a force is directed onto the contact glass (23) counter to the direction of incidence of the laser beam
**characterized in that** the safety mechanism is adapted to enable the moving apart by a bed (2) shiftable along the direction of incidence of the laser beam when a force exceeds a limit value of force (Fmin), which bed comprises by the headrest,
wherein the safety mechanism is designed to shift the bed (2) for moving apart and to fix the headrest (2) and the contact glass (23) to each other when force is below the limit value of force.

2. The laser treatment apparatus according to claim 1, **characterized in that** the safety mechanism is adapted to retract the contact glass (23).

3. The laser treatment apparatus according to claim 1 or 2, **characterized in that** the limit value of force (Fmin) is defined by an elastic force and/or a weight force.

4. The laser treatment apparatus according to claim 1, 2 or 3, **characterized in that** the contact glass (23) is mounted to a holding element (24) which is pressed against a stop of the housing (B, 6) with a force defining the limit value of force.

5. The laser treatment apparatus according to claim 4, **characterized in that** the holding element (24) also carries focusing optics (20) focusing the treatment laser beam (L) into or onto the eye (21).

6. The laser treatment apparatus according to any of the above claims, **characterized in that** the limit value of force is 1 Newton.

7. The laser treatment apparatus according to claim 1 comprising a beam-deflecting unit (15, 16) which variably deflects the treatment laser beam (L) about at least one axis (S2) of deflection and a focusing optics (20) arranged subsequent to the beam-deflecting unit (15,16) which focusses the laser beam (L) along an optical axis into or onto the eye (21), wherein the contact glass (23) is arranged subsequent to the focusing optics (20).

8. The laser treatment apparatus according to claim 7, **characterized in that** the safety mechanism retracts the contact glass (23).

9. The laser treatment apparatus according to claim 8, **characterized in that** the contact glass (23), the focusing optics (20) and the beam-deflecting element (15, 16) are rigidly connected to form a unit and the safety mechanism comprises a longitudinal guide of the unit.

10. The laser treatment apparatus according to claim 8 or 9, **characterized in that** the light path of the laser beam (L) is folded at least once (18) after the entrance pupil of the focusing optics (20) and that the safety mechanism effects a joint rotary movement or pivotal movement of the contact glass (23), the focusing optics (20), and the deflecting element (15) during retraction.

11. The laser treatment apparatus according to claim 10, **characterized in that** the contact glass (23), the focusing optics (20), and the deflecting element (15) are rigidly connected to form an arm and the safety mechanism comprises a rotary support (25) for the arm with an axis of rotation lying in a plane of the entrance pupil.

12. The laser treatment apparatus according to any of claims 8 to 11, **characterized in that** the safety mechanism comprises a weight force compensating unit (27, 28), especially in shape of a balancing weight or a spring element (27, 41).

13. The laser treatment apparatus according to claim 12, **characterized in that** the weight force compensating unit sets the limit value of force.

14. The laser treatment apparatus according to any of claims 7 to 13, **characterized by** a supporting unit (34, 36) which comprises a support (34) placeable in contact with the patient's body (33) and which is coupled to the safety mechanism such that a certain force acting on the support (34) counter to the direction of incidence of the laser beam also causes said moving apart.

## Revendications

1. Appareil de traitement laser destiné à la chirurgie oculaire, comportant un équipement de tête (2) destiné à un patient et un verre de contact (23) pouvant être placé sur l'oeil (21), à travers lequel passe un faisceau laser de traitement (L), dans lequel il est prévu un mécanisme de sécurité (24, 25) qui est conçu pour écarter l'un de l'autre l'équipement de tête (2) et le verre de contact (23) lorsqu'une force orientée en sens opposé à la direction d'incidence du faisceau laser est exercée sur le verre de contact (23),
**caractérisé en ce que** le mécanisme de sécurité est conçu pour permettre ledit mouvement d'écartement lorsque la force se situe au-dessus de la valeur limite de la force (Fmin) par l'intermédiaire d'un lit (2) qui est réglable dans la direction d'incidence du faisceau laser et qui comporte l'équipement de tête, dans lequel le mécanisme de sécurité est conçu pour régler le lit (2) afin de produire ledit mouvement d'écartement et, lorsque la force se situe en-dessous de la valeur limite de la force, pour fixer l'un à l'autre l'équipement de tête (2) et le verre de contact (23).

2. Appareil de traitement laser selon la revendication 1, **caractérisé en ce que** le mécanisme de sécurité est conçu pour rétracter le verre de contact (23).

3. Appareil de traitement laser selon la revendication 1 ou 2, **caractérisé en ce que** la valeur de force limite (Fmin) est exercée par une force de rappel et/ou pondérale.

4. Appareil de traitement laser selon la revendication 1, 2 ou 3, **caractérisé en ce que** le verre de contact (23) est fixé à un élément de maintien (24) qui est repoussé vers une butée du boîtier (B, 6) avec une force définissant la valeur limite de la force.

5. Appareil de traitement laser selon la revendication 4, **caractérisé en ce que** l'élément de maintien (24) porte également une optique de focalisation (20) qui focalise le faisceau laser de traitement (L) dans ou sur l'oeil (21).

6. Appareil de traitement laser selon l'une quelconque des revendications précédentes, **caractérisé en ce que** la valeur limite de la force est de 1 Newton.

7. Appareil de traitement laser selon la revendication 1, comportant une unité de déviation de faisceau (15, 16) qui dévie de manière variable le faisceau laser de traitement (L) au moins autour d'un axe (S2), et une optique de focalisation (20) qui est disposée en aval de l'unité de déviation de faisceau (15, 16) et qui focalise le faisceau laser (L) le long d'un axe optique dans ou sur l'oeil (21), dans lequel le verre de contact (23) est disposé en aval de l'optique de focalisation (20).

8. Appareil de traitement laser selon la revendication 7, **caractérisé en ce que** le mécanisme de sécurité rétracte le verre de contact (23).

9. Appareil de traitement laser selon la revendication 8, **caractérisé en ce que** le verre de contact (23), l'optique de focalisation (20) et l'unité de déviation de faisceau (15, 16) sont reliés de manière rigide en formant une unité et **en ce que** le mécanisme de sécurité comporte un guide longitudinal pour ladite unité.

10. Appareil de traitement laser selon la revendication 8 ou 9, **caractérisé en ce que** le chemin optique du faisceau laser (L) est dévié (18) au moins une fois après la pupille d'entrée de l'optique de focalisation (20) et **en ce que** le mécanisme de sécurité provoque lors de la rétraction un mouvement de rotation ou de pivotement du verre de contact (23), de l'optique de focalisation (20) et de l'élément de déviation (15).

11. Appareil de traitement laser selon la revendication 10, **caractérisé en ce que** le verre de contact (23), l'optique de focalisation (20) et l'élément de déviation (15) sont reliés de manière rigide à un bras et **en ce que** le mécanisme de sécurité présente un coussinet (25) de rotation du bras dont l'axe de rotation se situe dans le plan de la pupille d'entrée.

12. Appareil de traitement laser selon l'une quelconque des revendications 8 à 11, **caractérisé en ce que** le mécanisme de sécurité comporte un dispositif de compensation de force pondérale (27, 28), notamment sous la forme d'un contrepoids ou d'un élément à ressort (27, 41).

13. Appareil de traitement laser selon la revendication 12, **caractérisé en ce que** le dispositif de compensation de force pondérale règle la valeur limite de la force.

14. Appareil de traitement laser selon l'une quelconque des revendications 7 à 13, **caractérisé par** un dispositif de support (34, 36) qui comporte un support (34) pouvant être appliqué sur le corps (33) d'un patient et qui est couplé au mécanisme de sécurité de manière à ce qu'une force déterminée agisse sur le support (34) en sens opposé à la direction d'incidence du faisceau laser ainsi qu'au mouvement d'écartement.
